# EUROPEAN PATENT APPLICATION

(11) **EP 0 872 559 A1**
(43) Date of publication of application: **21.10.1998**
(21) Application number: 96937543.5
(22) Date of filing: 08.11.1996
(51) Int. Cl.: C12Q 1/68

(54) **METHOD FOR THE SIMULTANEOUS DETECTION OF POLYGENES**

(30) Priority: 09.11.1995 JP 317160/95
(71) Applicant: SHIONOGI & CO., LTD., Osaka-shi, Osaka-fu 541 (JP)
(72) Inventor: SUZUKI, Harukazu, Katano-shi, Osaka 576 (JP); YAOI, Takeshi, Toyonaki-shi, Osaka 560 (JP); WATANABE, Sachihiko, Izumi-shi, Osaka 590-02 (JP)
(74) Representative: Kügele, Bernhard
(86) International application number: JP9603289
(87) International publication number: WO9717466

(57) **Abstract**

A process for the simultaneous detection of multiple genes which comprises following steps ( a ) to ( f ) ;
( a ) a process for preparing cDNA from mRNA by using a primer for reverse transcription which has the following characteristics ( 1 ) to ( 3 ) ;
   ( 1 ) it has an anchor at the 3'-end of the oligo dT. The anchor is represented by the formula 5 ' -M(N)q-3 ' wherein M represents A, G or C and N represents A, G, C or T and q is 0 or 1;
   ( 2 )it has a portion for immobilization at the 5'-end of the oligo dT;
   ( 3 ) it has a site for enzyme digestion between the 5'-end of the oligo dT and the portion for immobilization ;
( b ) a process for digesting the cDNA species by a restriction enzyme,
( c ) a process for labeling the digested site of the cDNA fragment,
( d ) a process for recovering the labeled cDNA fragment by immobilizing it on a solid phase,
( e ) a process for digesting cDNA fragment at the site for enzyme digestion located at the 5'-end of the primer for reverse transcription to obtain the labeled cDNA fragment in the aqueous phase, and
( f ) a process for applying the labeled cDNA fragment to two-dimensional gel electrophoresis.

## Description

### Field of the Invention

The present invention relates to a process for the highly sensitive, quantitative and simultaneous detection of many kinds of cDNA species based on the two- dimensional electrophoresis, and to a process for the preparation of samples in which cDNA species for each mRNA species are uniform in length for the two-dimensional electrophoresis, and to a primer for reverse transcription which is useful for the preparation of the cDNA samples. cDNA fragments can be easily cloned from all gel spots detected by the two-dimensional gel electrophoresis of the present invention by an established method, e.g., Suzuki et al, DNA Res.1(1994)175-180., Hirotsune et al, Biochem. Biophys. Res. Commun. 194 (1993) 1406-1412. Therefore, the present invention allows the two-dimensional detection of the differentially expressed genes in the different type of cells or tissues and cells or tissues under the different conditions, and allows to examine where and what gene is expressed or not on development, differentiation, or aging. Further, the present invention allows to detect the difference in gene expression between morbid parts and normal parts, and the change of gene expression under the different condition by the particular pharmaceuticals etc. Additionally, the present invention allows the easy isolation of a novel gene which is useful for a diagnosis, a therapy including a gene therapy and a screening for pharmaceuticals, etc.

### Background of the Invention

In molecular biology, it is important to identify and analyze genes whose expression levels and patterns are different in various cell types, tissues, developmental stages or particular conditions. Differential hybridization "St. John et al, Cell 16 (1979) 443-452" and Subtraction hybridization "Zimmerman et al, Cell 21 (1980) 709-715" have been frequently used to detect and isolate such differentially expressed genes (J. Sambrook, E. F. Frisch, T. Maniatis, Molecular Cloning second edition, Cold Spring Harbor Laboratory Press 1989). Differential hybridization is easy and reliable but only effective for mRNAs expressed abundantly in one of two samples. Subtractive hybridization is effective for the concentration of rare mRNA species but is rather empirical and poor in reproducibility. Further, it has been pointed out that both methods take too much time (Liang and Pardee, Science 257 (1992) 967-971).

Recently, Liang and Pardee developed a display system called differential display (Science 257 (1992) 967-971). In this method, reverse-transcription PCR is performed with a combination of anchored oligo(dT) primers and arbitrary primers, and the PCR products are separated on a sequencing gel to detect differences in banding patterns due to differential transcription. Compared with the subtractive and differential hybridizations, the differential display is advantageous with respect to sensitivity, simplicity and time requirement. However, PCR-mediated amplification with arbitrary primers often gives false-positive bands that can not detect signals in RNA blotting analysis. Further, delicate control of amplification cycles and/or annealing temperature is necessary to detect differences in gene expression levels among samples. Furthermore, it may sometimes be difficult to detect differences in banding patterns due to differential transcription, since difference in the intensity of the corresponding bands between two samples does not always correlate quantitatively with difference in the expression level of the corresponding genes.

Hayashizaki et al. have recently developed a novel method, designated RLGS (restriction landmark genomic scanning), for systematic analysis of genomic DNA (Hatada et al, Proc. Natl. Acad. Sci. USA, 88 (1991) 9523-9527 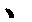 Hayashizaki et al, Electrophoresis,14 (1993) 251-258, JP-3-53899-A and JP-3-206897-A) . The principle of RLGS is based on using restriction enzyme sites as landmarks and two-dimensional gel electrophoresis with high resolution. This method has the following characteristics. ( 1 ) It allows to display 2000 to 3000 genomic loci as gel spots simultaneously. ( 2 ) It allows to display other genomic loci by using alternate restriction enzymes. ( 3 ) The intensity of spots reflects copy number of the DNA fragment. A haploid genomic fragment can be easily distinguished from a diploid fragment by its signal intensity. ( 4 ) It can be applied to any organism, because direct labeling of restriction sites (not hybridization procedure) is employed as the detection system. ( 5 ) It allows to scan CpG islands by using CpG-rich restriction enzymes. Further, the methylation status of CpG islands on genomic DNA can be examined by using a methylation-sensitive restriction enzyme. Thus, RLGS is a very powerful technique which has been applied to genetic mapping (Hayashizaki et al., Genetics 138 (1994) 1207-123, Okazaki et al., Proc. Natl. Acad. Sci. USA. 92 (1995) 5610-5614), search for aberration of cancer DNA (Hirotsune et al., Cancer Res. 52 (1992) 3642-3647, Nagai et al., Cancer Res. 54 (1994) 1545-1550, Miwa et al., Electrophoresis, 16 (1995) 227-232) and systematic detection of methylatable loci (Hayashizaki et bal., Electrophoresis, 14 (1993) 251-258, Kawai et al., Nucleic Acids Res. 21 (1993) 5604-5608, Kawai et al., Mol. Cell. Biol. 14 (1994) 7421-7427). Furthermore, techniques to clone the target DNA fragments from RLGS gel spots have also been established (Hirotsune et al., Biochem. Biophys. Res. Commun. 194 (1993) 1406-1412, Suzuki et al., DNA Res. 1(1994) 175-180, Watanabe et al., Electrophoresis, 16 (1995) 218-226). Actually, genes which correspond to specified spots were identified and isolated (Kawai et al., Biochem. Biophys. Res. Commun. 213 (1995) 967-974, Hayashizaki et al., Nature Genet. 6 (1994) 33-40) .

It has been thought that the RLGS method is so powerful for the analysis of genomic DNA that application of the RLGS system to the cDNA analysis should be useful. Nevertheless, it has not been reported that the RLGS system is applied to the cDNA analysis. This is because there are some problems which should be solved. One of the most serious problems was how to prepare cDNA species of uniform length for each mRNA species to detect each cDNA species as a gel spot. When cDNA is synthesized from a particular mRNA species in vitro by reverse-transcriptase, length of the synthesized cDNA species are heterogeneous due to the various starting and/or stopping site of reverse transcription. Therefore, each mRNA species may be detected as more than one gel spots and for many spots corresponding to each mRNA species may be noisy and make the analysis difficult.

The objects of the present invention are to provide a method that every cDNA for a particular mRNA species is prepared to be uniform in length and to provide a system for simultaneous detection of many cDNA species using the RLGS system.

### Disclose of the Invention

As a result of the research to solve the problems described above, we used an oligo(dT) anchor primer for reverse-transcription which are characterized by the following ( 1 ) to ( 3 ) . Because the primer is designed to anchor 5'-terminus of poly (A) tail site of mRNA, the starting site of reverse transcription become to be uniform for each mRNA species. Further, it is designed to recover easily the cDNA fragments in which each cDNA species are uniform in length and are labeled at the enzyme digestion site.

The present invention provides a process for the simultaneous detection of multiple genes which consists of the following steps ( a ) to ( f ) ; a process for preparing cDNA species of uniform length for each mRNA species which consists of the following steps ( a ) to ( f ) ; and a primer for reverse-transcription which has the following characteristics ( 1 ) to ( 3 ) ;
( a ) a process for preparing cDNA from mRNA by using a primer for reverse transcription which has the following characteristics ( 1 ) to ( 3 ) ;
   ( 1 ) it has an anchor at the 3'-end of the oligo dT. The anchor is represented by the formula 5' -M(N)q- 3' wherein M represents A, G or C and N represents A, G, C or T and q is 0 or 1;
   ( 2 )it has a portion for immobilization at the 5'-end of the oligo dT;
   ( 3 ) it has a site for enzyme digestion between the 5'-end of the oligo dT and the portion for immobilization ;
( b ) a process for digesting the cDNA species by a restriction enzyme,
( c ) a process for labeling the digested site of the cDNA fragment,
( d ) a process for recovering the labeled cDNA fragment by immobilizing it on a solid phase,
( e ) a process for digesting cDNA fragment at the site for enzyme digestion located at the 5'-end of the primer for reverse transcription to obtain the labeled cDNA fragment in the aqueous phase, and
( f ) a process for applying the labeled cDNA fragment to two-dimensional gel electrophoresis.

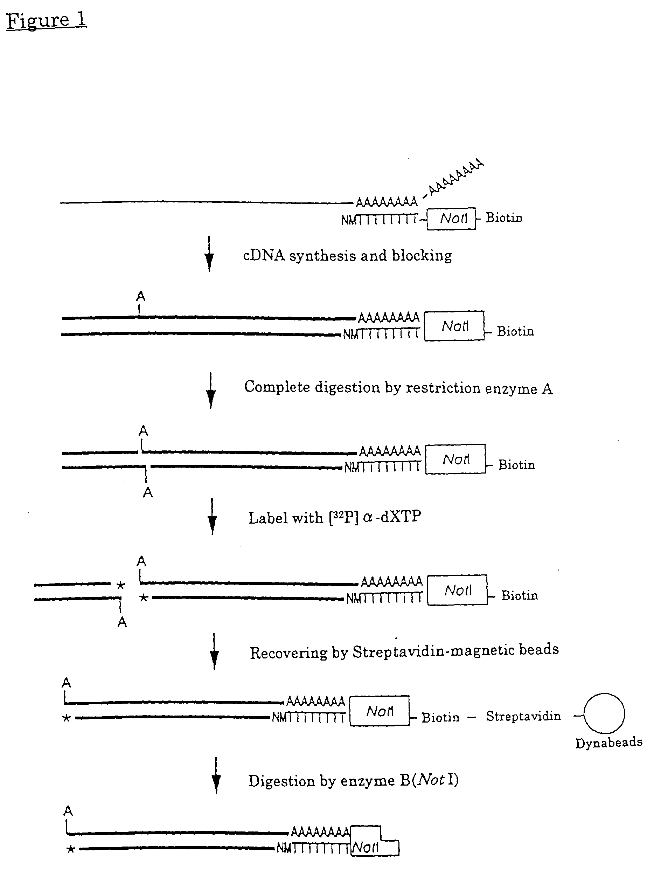

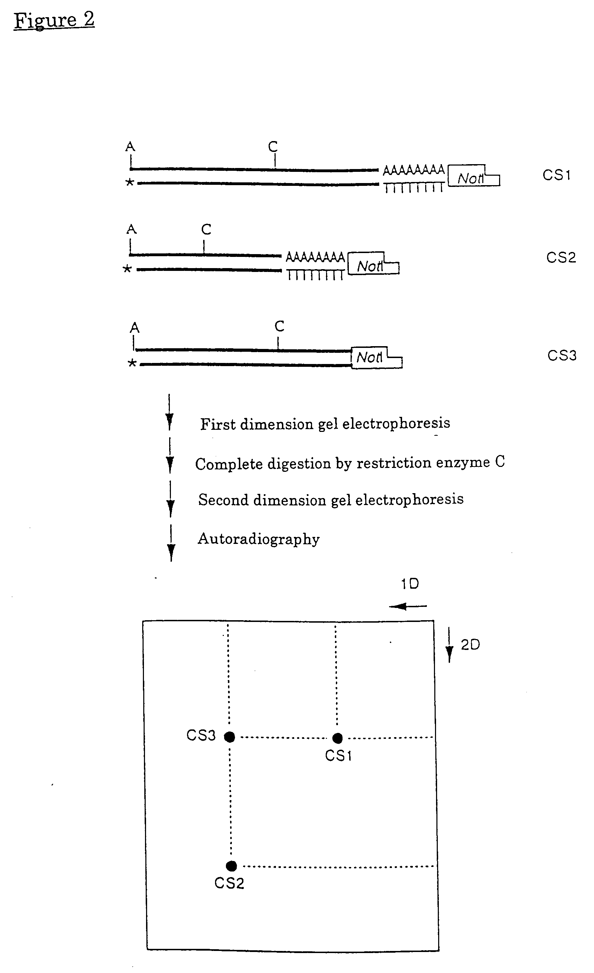

### 1 . Detail description of each steps(see also Figure 1 and Figure 2)

### ( a ) The step of preparing cDNA from mRNA

The present invention can be applied to any organisms that have mRNA with a poly (A) tail. It is enough that common methods to each organism are used to prepare mRNA. cDNA can be synthesized by a normal method, but it is necessary to use the primer which is designed for the present invention. The primer has the following characteristics ( 1 ) to ( 3 ) .
(1) The primer has an additional nucleotides M(N)q at the 3'-end of the oligo(dT) wherein M represents A, G or C and N represents A, G, C or T and q is 0 or 1. It is designated to anchor at the upstream end of the poly(A) tail, so as to make the poly(A) tail of the cDNA uniform in length. The number and the type of base represented by MN and the number of T in oligo(dT) can be appropriately decided by referencing to such papers (Khan et al., Nucleic Acid Res. 19 (1991) 1715, Liang et al, Nucleic Acid Res. 22 (1994) 5763-5764). Twelve to twenty T residues are desirable for the oligo(dT).
( 2 ) The primer has a portion for immobilization at the 5'-end to recover the final product easily. The portion for immobilization is an added portion for catching a target cDNA and recovering it at solid phase condition. Biotin, cellulose, magnet bead etc. are used for this purpose. When biotin is used, the synthesized cDNA can be easily recovered under the solid phase condition by using an immobilized Streptavidin. Further, when cellulose or magnet bead is used, the synthesized cDNA can be recovered under the solid phase condition by using itself as a solid phase. Among them, biotin is specially preferred.
( 3 )The primer has at least one appropriate enzyme site between the oligo(dT) and the portion for immobilization to recover cDNA easily from a solid phase condition. The site should be specifically digested by an enzyme. For example, a restriction enzyme site or poly(dU) for uracil DNA glycosylase can be used. The restriction enzyme site should be rare cutter site. For example, the restriction enzyme sites like *Not*I, *Spe*I, *Xba*I and *Nru*I can be used. For an enough effect of the enzyme, it is preferred that the enzyme digestion site is 5 to 15 bases apart from the portion for immobilization. And it is more preferred that more than two enzyme sites are designed, because the enzyme can appropriately be changed.

A concrete example of the primer with the characteristics described above is as follows.
5' -biotin-ACTAGTTCTAGATCGCGAGCGGCCGCCC(T)₁₄TMA-3'
(wherein, M represents A, G or C and the underline shows restriction enzyme site *Not*I. The restriction enzyme sites, *Soe*I, *Xba*I and *Nru*I, are also designed between biotion and *Not*I site)

The example described above can be made by referencing commercialized *Not*I-Primer-Adaptor (Gibco BRL). A mixture of the above primers with M as A, G or C (the mixing ratio ; A : G : C= 1 : 1 : 1) is designated biotinylated anchor primer BDT02A.

Actually at the cDNA synthesizing reaction, after mixing 1 µg of the described anchor primer and several µg of poly(A) + RNA , heating it for 5 to 15 minutes (10 minutes to be preferred) at 60 to 80°C (70°C to be preferred) and getting it cool on ice, double stranded cDNA can be synthesized by commercialized cDNA synthesizing kits (e.g. Super Script ™ Lambda System (Gibco BRL)). An synthesized cDNA can be recovered by ethanol-precipitation.

On the present invention, it is preferred that the remained mRNA is excluded after the cDNA synthesis. Because, the present inventor found out that the remained mRNA is cause of undesirable noise on two-dimensional electrophoresis display. Therefore, it is necessary for a good result to exclude the remained mRNA.

Any methods which exclude RNA specifically can be applied for this purpose. Enzymatic digestion by RNase is preferred to exclude the remained mRNA . Any types of RNases can be adapted, but it must not have a DNase activity. It is preferred that the enzyme can digest RNA completely to mono-nucleotide, e.g. commercialized RNaseOne (Promega) or RNaseA (Sigma).

As a method for RNase treatment, the ethanol precipitate obtained from step ( a ) is dissolved in a suitable buffer solution for treatment with RNase at 20 to 45 °C (37 °C to be preferred) for 15 to 60 minutes (30 minutes to be preferred), followed by phenol-chloroform extraction. The aqueous layer is subjected to spin column treatment and cDNA in the eluate is recovered by ethanol precipitation.

In general, the synthesized cDNA often possesses several damages like nicks and gaps. Because these nicks and gaps are non-specifically labeled when particular restriction enzyme site is labeled for landmarking, it is worth repairing these damages on DNA to reduce non-specific labeling. Therefore, on the present invention, it is preferred that cDNA is repaired by blocking process after the remained mRNA is excluded. The method for blocking process is not limited if it is effective for the DNA repair. A reaction of ddNTP addition by Sequenase™ is an effective method. Concretely, blocking is performed in the presence of ddNTP and Sequenase™ at 20 to 42 °C (37 °C to be preferred) for 3 to 60 minutes (30 minutes to be preferred). After reaction, the mixture is treated with phenol/chloroform and the aqueous layer is subjected to a spin column followed by ethanol precipitation. It is preferred that excess of ddNTP and Sequenase™ should be used for the blocking reaction.

### ( b ) The step of digesting by a restriction enzyme

cDNA obtained from the above step is digested by appropriate restriction enzyme. In the present invention, a restriction enzyme used at the present step is designated " restriction enzyme A". It is desirable that restriction enzyme A is selected for easy labeling at the enzyme site of cDNA fragments. For this purpose, it is preferred that restriction enzymes which create protruding cohesive 5'-ends, e.g. *Bam*HI or *BgI*II, are used as the restriction enzyme A. But there is no needs to limit the restriction enzyme which create protruding cohesive 5'-ends. The restriction enzymes creating protruding cohesive 3'-ends or blunt ends can be used as restriction enzyme A if the resulting enzyme sites are specifically labeled.

Further, it is desirable to select the restriction enzyme A for getting 1000 to 2000 spots (approximately 1500 spots to be preferred) on the two dimensional gel. For this purpose, if it is necessary, it is allowed to use simultaneously more than two restriction enzymes as the restriction enzyme A.

As a method for proceeding, in the case of using *Bam*H I and *Bgl* II at the same time, cDNA equivalent to 1 µg starting poly(A) + RNA (normally 0.2 to 0.3 µg) is digested with excess of *Bam*H I and *BgI*II. The reaction mixture is treated with phenol/chloroform followed by spin column treatment and cDNA is recovered by ethanol precipitation.

### ( c ) The step of labeling

cDNA fragments are labeled by radio-isotope at the restriction enzyme A sites. When the cDNA fragment possesses the protruding cohesive 5'-end, labeling reaction can be performed with the enzyme like Sequenase™. When the cDNA fragment possesses the protruding cohesive 3'-end or blunt end, labeling reaction can be performed with the enzyme like a terminal nucleotide transferase. For example, the protruding cohesive 5'-end of cDNA fragment can be labeled with Sequenase™ as follows. When *Bam*H I and *BgI*II are used as the restriction enzyme A, cDNA fragments are labeled with Sequenase™ in buffer solution containing ddCTP, ddTTP and [ α -³²P] dGTP at 20 to 42 °C (25 ° C to be preferred) for 3 to 15 minutes (5 minutes to be preferred). The reaction is terminated by EDTA. The reaction mixture is treated with phenol/chloroform and subjected to spin column treatment. At this labeling process, an unit number of Sequenase™ should be 0.5 to 5U (0.75U to be preferred). Since the non-specific labeling of the sample with Sequenase™ increased in parallel with the enzyme concentration, short reaction time should be selected in case of the reaction with high unit number of the enzyme.

### ( d ) The step for immobilization of labeled cDNA

Anchor primer's site for immobilization on cDNA fragment is reacted with solid phase, and the labeled cDNA fragment is immobilized and recovered. As described above, when biotin is used for the portion for immobilization, the labeled cDNA fragments are recovered by using Streptavidin fixed bead, ball, tube, etc. Magnetic beads are preferred as a solid phase because it can easily be recovered by using a magnet. Concretely, the labeled cDNA fragments are incubated with a solid phase in buffer solution with gentle rotation at 20 to 45 °C (37 °C to be preferred) for 10 to 60 minutes (30 minutes to be preferred). The solid phase is recovered and washed with an appropriate buffer solution, to obtain the immobilized cDNA fragments. When cellulose or magnetic beads is used for the site for immobilization of primer, there is no needs to react with a solid phase because it has already been become to be an immobilized condition. In this case it is enough to recover and wash the immobilized cDNA fragments.

### ( e ) The step to cleave off the site for immobilization

The cDNA fragments are cleaved off from the solid phase by enzyme digestion at anchor primer. The cDNA fragments in aqueous phase is recovered again. Each labeled cDNA species recovered should be theoretically uniform in length. This is the most important point in this invention. As a proceeding method, common methods can be appropriately proceeded. Concretely, when *Not* I is used for the enzyme digestion, the immobilized cDNA fragments are treated with excess of *Not* I (e.g. 10 to 40 U) at 20 to 42 °C (37 °C to be preferred) for 15 to 60 minutes (30 minutes to be preferred). After excluding the solid phase, the supernatant is treated with phenol/chloroform and cDNA is recovered by ethanol precipitation.

### ( f ) Two-dimensional gel electrophoresis

The sample is subjected to the two-dimensional gel system, in which the RLGS techniques and apparatus can be basically applied. RLGS can be performed by following the papers (Hatada et al., Proc. Natl. Acad. Sci. USA, 88 (1991) 9523-9527, Hayashizaki et al., Electrophoresis, 14 (1993) 251-258, Okazaki et al., Biochem. Biophys. Res. Commun. 205 (1994) 1922-1929, Hayashizaki et al., The Encyclopedia of Molecular Biology, Fundamental and Applications. VCH Inc., JP 3-53899-A and JP 3-206897-A) . As a concrete example, a 10 µl portion of cDNA sample recovered at the step described before is electrophoresed in a first dimension gel (1 % agarose gel) until bromophenol blue (BPB) migrates about 50cm. After electrophoresis, the gel is incubated with appropriate restriction enzyme buffer twice for 10 minutes and is treated with excess of restriction enzyme (the enzyme in this step is designated "restriction enzyme C". *Hin*f I is given as an example) at 20 to 42 °C (37 °C to be preferred) for 1 to 6 hours (2 hours to be preferred). The sample in the agarose gel is subjected to a second dimension polyacrylamide gel (6 %) electrophoresis.

### (g) The detection

The gel is normally dried after the two-dimensional gel electrophoresis. When the radioisotope is used for the labeling, spots can be detected by autoradiography. When non-radioisotope material is used for the labeling, the spots can be detected by the method which is suitable for the labeling material. Target DNA fragments corresponding to a particular spot, can be easily cloned by established methods (e.g. Suzuki et al., DNA Res. 1 (1994)175-180, Hirotsune et al., Biochem, Biophys. Res. Commun. 194 (1993) 1406-1412). Therefore, difference in the mRNA expression among samples can be confirmed by Northern blotting method using cloned DNA as a probe. Actually, it was demonstrated on the present invention that differences in intensity of the spot among profiles correlates well with expression levels of the corresponding mRNA species among samples. The sensitivity of the present invention can be estimated, according to the sensitivity of RLGS in which several thousands sites can be detected as spots on a gel, as follows. In RLGS, 1 µg genomic DNA, which is equivalent to 3 × 10⁵ copies of genomic DNA in mammalian cells, is usually used per analysis. Since the mainly observed spots on RLGS profile are originated from deploid genome, 3 × 10⁵ copies of labeled DNA fragments can be sufficiently detected as a spot. Because some spots with intensities one third weaker than those of the mainly observed spots are detectable on RLGS profiles (e.g. Suzuki, DNA Res.1 (1994) 175-180) , we estimate that at least 10⁵ copies of a labeled DNA fragment can be detected as a spot in the two-dimensional gel system. In RLCS analysis, when 1 µg poly(A) ⁺ RNA was used, 0.2 to 0.3 µg cDNA with an estimated mean size of 1000 bp was normally synthesized in our experiment (data not shown). Therefore, the total number of cDNA molecules is calculated as { (0.2 to 0.3) × 10⁻⁶} ÷ (660 × 1000 bp) × (6 × 10²³) = 0.18 to 0.27 × 10¹² (copies). Since the population of very rare mRNA species, whose expression level is approximately 1 copy / cell, is thought to be 0.0001 % of total mRNA (J. Sambrook, E. F. Frisch, T. Maniatis, Molecular Cloning second edition, Cold Spring Harbor Laboratory Press 1989), the number of cDNA corresponding to such mRNA species is calculated as 0.18 to 0.27 × 10¹² × 0.0001% = 1.8 to 2.7 × 10⁵. This value is comparable to the estimated limited number of 10⁵ copies for detection. Thus, very rare mRNA species may be detectable in the present invention.

### 2 . Other embodiment of the present invention

Because target genes for detection by this invention are not identified, it is not known what kind of restriction enzyme sites each target gene has. When more than two RLCS profiles, in which the product of step ( a ) is digested by more than two restriction enzymes separately at step ( b ) followed by steps ( c ) to ( f ) , are analyzed together, it is possible to detect more spots (genes) than those detected from single RLCS profile. But in this case, there is a disadvantage that some genes may be detected in more than two RLCS profiles. To avoid this redundancy, it is preferred to obtain more than two profiles by the following steps. After labeling in step ( c ), sample is digested by the restriction enzyme(s), which is used for other profiles in step ( b ) , followed by steps ( d ) to ( f ) .

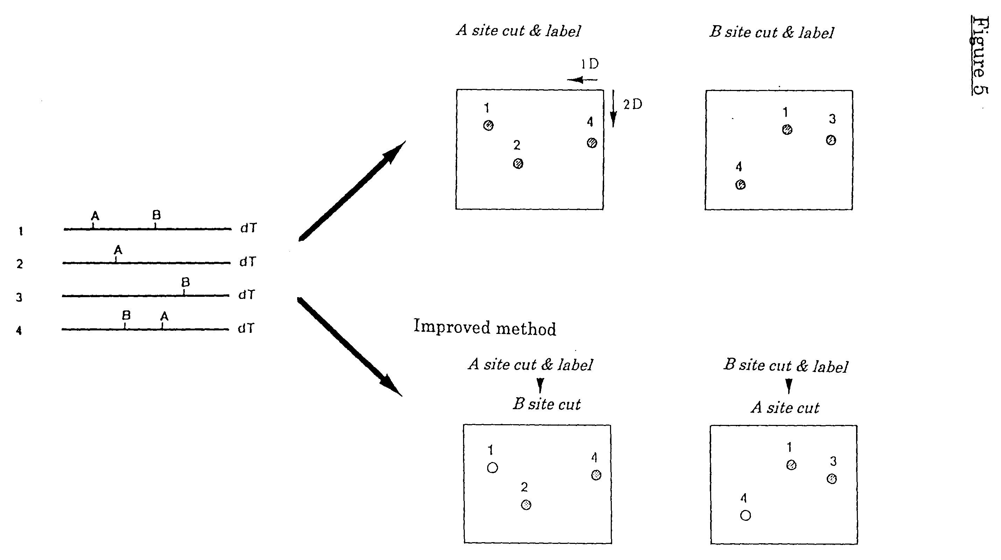

For example, the case for obtaining two profiles by using two restriction enzymes A and B, is explained in Fig. 5. Each cDNA species are classified into 4 groups by existence of the restriction enzyme sites A and B. When cDNAs are treated separately with two restriction enzymes A and B, cDNA species in groups 1 and 4 are displayed in both profiles as spots 1 and 4 , respectively. To avoid this redundancy, after the digestion by restriction enzyme A in step ( b ) and the labeling in step ( c ) , sample is digested by restriction enzyme B followed by two-dimensional electrophoresis. In the same way, after the digestion by restriction enzyme B in step ( b ) and the labeling in step ( c ) , sample is digested by restriction enzyme A followed by two-dimensional electrophoresis. Thus, the redundancy between two profiles is excluded. This principle can be adapted for using more than three restriction enzymes. That is to say, first profile is obtained from sample which has a treatment with mixture of enzymes (E2 to En) after the digestion by enzyme E1 and the labeling. Second profile is obtained from sample which has a treatment with mixture of enzymes (E1 and E3 to En) after the digestion by enzyme E2 and the labeling. In this way, N piece of profiles without any spot redundancies, can be obtained.

The restriction enzymes used for the purpose described above are preferred to be satisfied with the conditions which are described in step ( b ) . Further, when mixture of more than two restriction enzymes are used for the first digestion in step ( b ) , it is necessary that these enzymes should not be other enzymes that are used in step ( b ) for other RLCS profiles.

### Example

The present invention is explained more concretely by the following examples. However, it does not limit the present invention.

### Example 1

Liver and brain were isolated from 7 week old mice. The brain was separated into 3 regions, cerebral cortex, cerebellum and brain stem. Total RNA was prepared from the tissues by Acid Guanidinium Thiocyanate-Phenol-Chloroform (A G P C) method (P. Chomczynski and N. Sacchi, Anal. Biochem. 162 (1987) 156-159) . Poly(A) + RNA was purified from total RNA with Oligotex-dT30 (Roche). Using this RNA, cDNA synthesis was performed as follows. 5.0 µg of liver poly(A)+ RNA or 3.4 µg of poly(A) + RNA from each brain region and 1 µg of the biotin-anchor primer BDT02A were mixed and heated to 70 °C for 10 minutes, and chilled on ice. Double-stranded cDNA was synthesized with the commercialized cDNA synthesizing kit (Super Script ™ Lambda System; Gibco-BRL) . After the cDNA synthesis, the reaction mixture was treated with phenol/chloroform followed by ethanol precipitation. The yield of double stranded cDNA was 0.2 to 0.3 µg from 1 µg poly(A) + RNA at this step. The precipitate was dissolved in 70 µl of buffer solution A (50mM Tris-HCl pH7.4, 10mM MgCl₂, 10mM DTT) for treatment with RNaseA (20 µg/ml at final concentration) at 37 °C for 30 minutes, followed by phenol/chloroform extraction. The aqueous layer was subjected to spin column treatment (CHROMA SPIN-100, Clontech), and cDNA in the eluate was ethanol-precipitated. The precipitate was incubated in 25 µl buffer solution A in the presence of each 100 µM of ddNTPs and 6.5 units of Sequenase™ at 37 °C for 30 minutes. After the reaction, the mixture was treated with phenol/chloroform. The aqueous layer was subjected to spin column treatment followed by ethanol precipitation.

The precipitate was dissolved in buffer solution A. cDNA equivalent to 1 µg of starting poly(A) + RNA was digested in 50 µl with *Bam*H I (19 units) and *BgI* II (16 units) at 37 °C for 60 minutes. The reaction mixture was treated with phenol/chloroform and cDNA was ethanol-precipitated. Labeling of the digested cDNA at the restriction enzyme sites was done in 10 µl of buffer solution A containing 100 µM ddCTP, 100 µM ddTTP, 50 µM [ α -³²P] dGTP(6000 Ci/mmole) and 1.4 unites of Sequenase™ at 22 °C for 5 minutes. The reaction was terminated by adding 25 mM EDTA at final concentration. The reaction mixture was treated with phenol/chloroform and subjected to spin column treatment. The eluate was incubated with 0.6 mg of Dynabeads M-280 streptavidin (DYNAL) in STE-BSA buffer solution (1 M NaCl, 10 mM Tris-HCl pH 8.0, 1 mM EDTA and 0.01 % BSA) with gentle rotation at 37 °C for 30 minutes. The Dynabeads were then collected magnetically, and washed once with STE-BSA buffer solution and twice with *Not* I buffer solution (50mM Tris-HCl pH7.5, 10mM MgCl₂, 1mM DTT and 100mM NaCl). Next, the Dynabeads were incubated with excess units (30 units) of *Not* I at 37 °C for 30 minutes. After removing the Dynabeads, the supernatant was treated with phenol/chloroform and then subjected to ethanol precipitation. The precipitate was dissolved in 10 µl of TE.

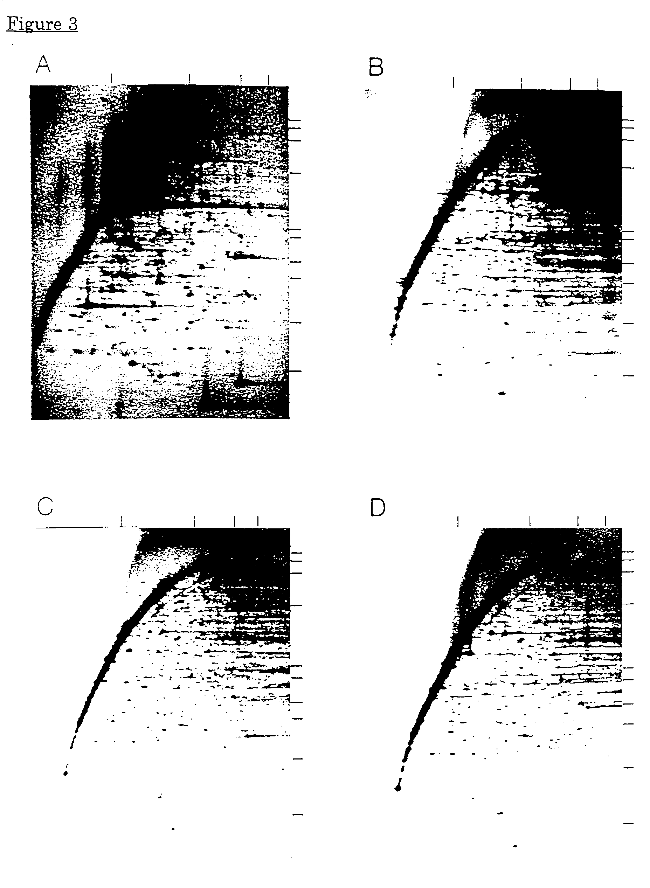

Two-dimensional gel electrophoresis was performed by following the reference papers (Hayashizaki et al., The Encyclopedia of Molecular Biology, Fundamental and Applications. VCH Inc. in press., Okazaki et al., Electrophoresis 16 (1995) 197-202, Asakawa et al., Electrophoresis 16 (1995) 241-252, Hayashizaki et al., A Practical Course on the Restriction Landmark Genomic Scanning (RLGS) Method (RLGS Ver. 1.8), Hayasizaki et al., The 16th annual meeting of the molecular biology society of Japan(1993), abstract No. 1319). Briefly, electrophoretic apparatus for the first-dimensional gel (vertical style using Teflon tube (Sanplatec Corp., Osaka, Japan) with inner diameter of 2.4mm) and second-dimensional gel (4 flat plates gel type) was purchased from Biocraft Inc., Japan. A vertical disc gel (1 % agarose gel (SeaKem GTC, FMC Corp.) ) in Teflon tubing was used for the first-dimensional gel. A 10 µl portion of RLCS sample was electrophoresed to the gel until bromo-phenol blue (BPB) migrated approximately 50 cm (300 V, 15 hours). After the electrophoresis, the gel rod was taken out from the Teflon tubing and was incubated with *Hin*f I buffer twice for 10 minutes followed by treatment with 750 units of *Hin*f I at 37 °C for two hours. The sample in the agarose gel rod was then subjected to second-dimensional gel electrophoresis at 180V for 15 hours (6 % polyacrylamide (NACALAI TESQUE Inc., Kyoto, Japan)). After the second-dimensional gel electrophoresis was performed, the polyacrylamide gel was dried and autoradiographed at -70 °C. The result is shown in Figure 3.

### Example 2

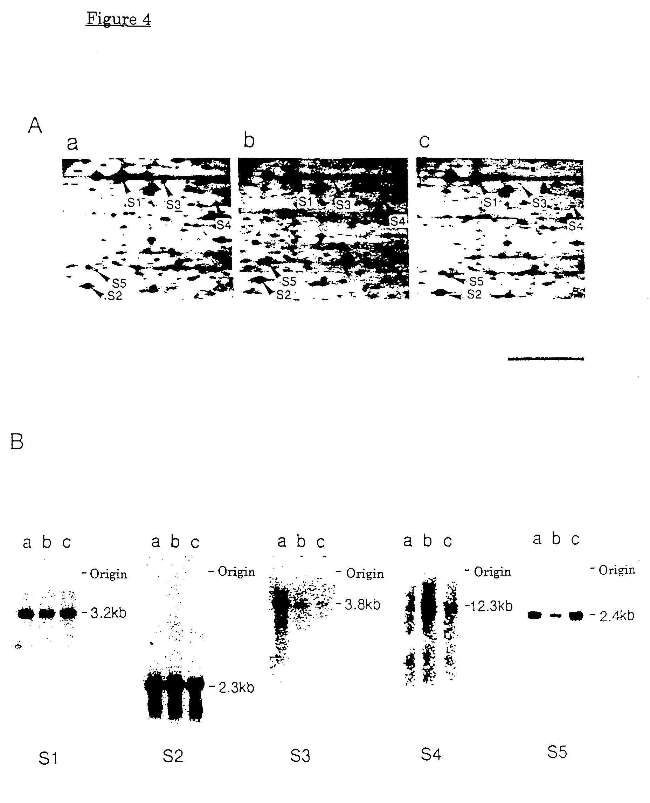

The RLCS profiles among three brain regions ( Fig. 3B, 3C and 3D) are so similar that the corresponding spots among the brain regions can be identified. We tried to clone cDNA fragments from several spots; spots S1 and S2 in Fig. 4A, whose intensities are almost same among three brain regions and spots S3, S4 and S5 in Fig. 4A, whose intensities are different among three brain regions. Cloning of the cDNA fragments from each spot was basically performed by following the paper (Suzuki et al., DNA Res. 1 (1994) 175-180). The electro-eluted target spot DNAs were ligated with *Bam*H I and *Hin*f I adapters that consist of pre-annealed double-stranded oligonucleotides: 5'-CGCCAGGGT TTTCCCAGTCACGACG-3' and 5'-pATCCGTCGTGACTGGGAAAACC CTGGCG-3' for the *Bam*H I site and 5'-CGCCAGGGTTTTCCCAGT CACGACG-3' and 5'-pANTCGTCGTGACTGGGAAAACCCTGGCG-3' for the *Hin*f I site. The spot DNAs were then purified with the spin column, and PCR-amplified (94 °C 1 min., 60 °C 1.5 min., 72 °C 2 min. for 20 to 30 cycles) using a M13 forward HT primer, 5'-CGCCAGGGTTTTCCCAGTCACGACG-3'. The amplified DNA fragments were purified using polyacrylamide gel and the Wizard PCR Preps DNA Purification System (Promega). The purified fragments were digested with *Bam*H I and then ligated with a *Bam*H I -dT vector, which was prepared from pT7 Blue T-vector (Novagen) by *Bam*H I digestion, followed by transformation into E. coli. Fig. 4B shows the result of RNA blotting analysis using the cloned spot DNAs as probes. Poly(A) + RNA derived from each tissue was denatured by formaldehyde and electrophoresed to agarose gel. RNA was blotted onto Hybond-N + nylon membrane (Amersham). Hybridization was performed with Quik-Hyb (Stratagene).

### Example 3

The rat pheochromocytoma cell line PC12 (L. A. Greene and A. S. Tischler, Proc. Natl. Acad. Sci. U. S. A. 73 (1976) 2424 - 2428) was cultivated (medium: DMEM containing 10 % FCS) by the following common method (see, e.g., Seno,K., Koyama,H., and Kuroki,T., Animal cell manual ; Kyoritsu Publishing Co.(1993), Endo,H., Animal cell culture; R&D Planning(1985)etc.) and approximately 5 × 10⁷cells were obtained. cDNA was prepared from these cells by the method described in example 1. Three enzymes , *Eco*R I(E), *Hin*d III (H) and *Nco* I(N) were used as restriction enzyme A. That is to say, first-dimensional gel electrophoresis was performed for the first sample that was treated by enzyme E and labeled followed by treatment by other two enzymes (H and N). In the same way, first-dimensional gel electrophoresis was performed for the second and third samples that were treated in respectively by enzyme H and N and labeled followed by treatment by other two enzymes (in respectively enzymes E · N and E · H). After the first-dimensional gel electrophoresis, each gel was treated by *Hin*fI and subjected to the second-dimensional gel electrophoresis followed by autoradiography for the spot detection. Three profiles were obtained from this experiment and the number of gel spots observed on each profile was 1050, 1161 and 985 (on the line of 'double digestion' in Table 1). As there was essentially no redundancy among the gel profiles, it means that the total of 3196 independent genes were displayed. On the other hand, when the conventional RLCS was performed using enzymes E, N and H separately (in the line of 'no digestion' in Table 1), 1332, 1634 and 1578 spots were detected, respectively. However, gel spots (genes) from only one profile could be counted without redundancy. Consequently, it was suggested that the present invention is useful for displaying many kinds of genes without redundancy.

**Table 1**

| | *Eco*RI label | *Nco*l label | *Hind*III label |
|---|---|---|---|
| no digestion | 1332 | 1634 | 1578 |
| double digestion | 1050 | 1161 | 985 |

### Effect of the present invention

Following the present invention, multiple genes can be simultaneously and quantitatively detected as gel spots with high sensitivity and without redundancy. Thus, difference in intensity of the corresponding spot reflects expression level of the corresponding gene, and the present invention has high sensitivity to detect mRNA species whose expression level is very low. Further, this invention can be adapted to any organism, and it is also possible to clone target DNA from all spots. Therefore, this invention enables us to provide an effective and excellent method to detect and clone genes which is expressed in only one of the two different types of cells or cells under different conditions.

### Figure legend

### Figure 1

Figure 1 shows a preparation method of cDNA sample in which cDNA species of uniform length are prepared for each mRNA species. Thin horizontal line and thick doubled horizontal lines indicate poly(A) + RNA and double-stranded cDNA, respectively. Asterisks show the radio-labeled nucleotides incorporated.

### Figure 2

Figure 2 shows a separation of cDNA species by two-dimensional gel electrophoresis. cDNA fragments in an RLCS sample (CS1, CS2 and CS3) are separated by two-dimensional gel electrophoresis and detected as spots CS1, CS2 and CS3, respectively.

### Figure 3

A : a photograph which shows RLCS profile using cDNA sample from mouse liver.
B : a photograph which shows RLCS profile using cDNA sample from mouse cerebral cortex.
C : a photograph which shows RLCS profile using cDNA sample from mouse cerebellum.
D : a photograph which shows RLCS profile using cDNA sample from mouse brain stem.

A thick horizontal bar means 10 cm. Scales in each photograph are 1.88, 1.49, 0.93 and 0.42 kb (from right to left) and 1357, 1078, 872, 603, 310, 281/271, 234, 194, 118 and 72 bp (from up to down) for first-and second-dimensions, respectively.

### Figure 4

A : photographs showing portion of RLCS profiles from mouse cerebral cortex ( a ) , cerebellum ( b ) and brain stem ( c ) . S1 and S2 show the spots whose intensities are almost same among three brain regions. S3, S4 and S5 show the spots whose intensities are different among three brain regions. A thick horizontal bar means 5 cm.
B : photographs showing the results of Northern blot analysis. cDNA fragments cloned from spots S1 and S2 in Fig. 4A, whose intensities are almost same among three brain regions, and spots S3, S4 and S5 in Fig. 4A, whose intensities are different among three brain regions, are used as probes for Northern blot analysis. Poly (A) + RNA from cerebral cortex, cerebellum and brain stem is used for lanes a , b and c , respectively. Differences in intensity of Northern signals among samples correlate well with intensities of the corresponding spots among RLCS profiles.

### Figure 5

Comparison between the conventional (upper) and improved (bottom) methods for this invention is shown. The spot patterns are compared between the conventional method, in which four types of unique cDNA species (1 to 4) with restriction sites A and/or B are digested with restriction enzyme A and B separately and labeled followed by two-dimensional electrophoresis, and the improved method, in which the cDNA species digested by restriction enzyme A and B separately and labeled, are digested by the other restriction enzyme followed by two-dimensional electrophoresis. Filled and open circles indicate spots which appeared and disappeared, respectively.

## Claims

1. A process for the simultaneous detection of multiple genes which comprises following steps ( a ) to ( f ) ;
( a ) a process for preparing cDNA from mRNA by using a primer for reverse transcription which has the following characteristics ( 1 ) to ( 3 ) ;
( 1 ) it has an anchor at the 3'-end of the oligo dT. The anchor is represented by the formula 5' - M(N)q- 3' wherein M represents A, G or C and N represents A, G, C or T and q is 0 or 1;
( 2 )it has a portion for immobilization at the 5'-end of the oligo dT;
( 3 ) it has a site for enzyme digestion between the 5'-end of the oligo dT and the portion for immobilization ;
( b ) a process for digesting the cDNA species by a restriction enzyme,
( c ) a process for labeling the digested site of the cDNA fragment,
( d ) a process for recovering the labeled cDNA fragment by immobilizing it on a solid phase,
( e ) a process for digesting cDNA fragment at the site for enzyme digestion located at the 5'-end of the primer for reverse transcription to obtain the labeled cDNA fragment in the aqueous phase, and
( f ) a process for applying the labeled cDNA fragment to two-dimensional gel electrophoresis.

2. The process of claim 1 wherein N is A and q is 1 in the base sequence of the formula : M(N)q.

3. The process of claim 1 wherein the portion for immobilization is biotin.

4. The process of claim 1 wherein a site for enzyme digestion is a restriction enzyme site.

5. The process of claim 1 which comprises an additional step for excluding the remained mRNA after ( a ) step.

6. The process of claim 5 wherein the remained mRNA is excluded by RNase.

7. The process of claim 6 which comprises an additional step for repairing cDNA by blocking treatment after the remained mRNA is excluded.

8. The process of claim 7 wherein the blocking treatment is performed using ddNTP.

9. The process of claim 1 which comprises an additional step after step ( c ) for digesting the cDNA fragment with one or more restriction enzyme(s) which is different from the restriction enzyme used in the step ( b ) .

10. The process of claim 1 in which the same gene can be detected without duplication, whereby that n kinds ( n is an integer of two or more) of the restriction enzymes ( E 1 - E n ) are used in the step ( b ) when the cDNA species obtained in the step ( a ) is applied to the steps ( b ) ~ ( f ) ,
which comprises the first step that the cDNA species obtained in the step step ( a ) is digested with the first enzyme ( E 1 ) ,the digested site is labeled, the labeled fragment is digested with all enzymes ( E 2 - E n) from which enzyme ( E 1 ) is excluded, and then the steps ( d ) ~ ( f ) are applied, to obtain the two-dimensional electrophoresis gel,
the second step that the cDNA species obtained in the step ( a ) is digested with the second enzyme ( E 2 ) , the digested site is labeled, the labeled fragment is digested with all enzymes ( E 1 , E 3 - E n ) from which enzyme ( E 2 ) is excluded, the steps ( d ) ~ ( f ) are applied, to obtained the two-dimensional electrophoresis gel, and the following steps in which the same applications are repeated except using the third enzyme ( E 3) to n the enzyme ( E n) , to obtain n species of the two-dimensional electrophoresis gel.

11. A process for preparing labeled cDNA species of uniform size which comprises the following steps ( a ) to ( e ) ;
( a ) a process for preparing cDNA from mRNA by using a primer for reverse transcription which has the following characteristics ( 1 ) to ( 3 ) ;
( 1 ) it has an anchor at the 3'-end of the oligo dT. The anchor is represented by formula : M(N)q wherein M represents A, G or C ; N represents A, G, C or T ; and q is 0 or 1 ;
( 2 ) it has a portion for immobilization at the 5'-end of the oligo dT;
(3) it has a site for enzyme digestion between the 5'-end of oligo dT and the portion for immobilization ;
( b ) a process for digesting the cDNA species by a restriction enzyme,
( c ) a process for labeling the digested site of the cDNA fragment,
( d ) a process for recovering the labeled cDNA fragment by immobilizing it on a solid phase,
( e ) a process for digesting the primer for reverse transcription at the site for enzyme digestion located at the 5'-end of the primer to obtain the labeled cDNA fragment in the aqueous phase.

12. A primer for reverse transcription which has the following characteristics ( 1 ) to ( 3 ) ;
( 1 ) it has an anchor at the 3'-end of the oligo dT. The anchor is represented by the formula : M(N)q wherein M represents A, G or C ; N represents A, G, C or T ; and q is 0 or 1.
( 2 ) it has a portion for immobilization at the 5'-end of the oligo dT.
( 3 ) it has a site for enzyme digestion between the 5'-end of the oligo dT and the portion for immobilization.
